(19) Europäisches Patentamt
European Patent Office
Office européen des brevets

(11) EP 0 500 853 B1

(12) **EUROPÄISCHE PATENTSCHRIFT**

(45) Veröffentlichungstag und Bekanntmachung des Hinweises auf die Patenterteilung:
**27.12.1996 Patentblatt 1996/52**

(21) Anmeldenummer: **91915303.1**

(22) Anmeldetag: **03.09.1991**

(51) Int Cl.[6]: **C07C 255/59**, A61K 31/275, A61K 31/36, A23K 1/16, C07C 271/28

(86) Internationale Anmeldenummer:
**PCT/EP91/01658**

(87) Internationale Veröffentlichungsnummer:
**WO 92/04316 (19.03.1992 Gazette 1992/07)**

(54) **PHENYLETHANOLAMINE, DIESE VERBINDUNGEN ENTHALTENDE ARZNEIMITTEL UND VERFAHREN ZU IHRER HERSTELLUNG**

PHENYLETHANOLAMINES, DRUGS CONTAINING THESE COMPOUNDS, AND A METHOD OF PREPARING THEM

PHENYLETHANOLAMINES, MEDICAMENTS CONTENANT CES COMPOSES ET LEUR PROCEDE DE PRODUCTION

(84) Benannte Vertragsstaaten:
**AT BE CH DE DK ES FR GB GR IT LI LU NL SE**

(30) Priorität: **07.09.1990 DE 4028398**

(43) Veröffentlichungstag der Anmeldung:
**02.09.1992 Patentblatt 1992/36**

(73) Patentinhaber: **Dr. Karl Thomae GmbH**
**D-88397 Biberach (DE)**

(72) Erfinder:
- **PIEPER, Helmut**
  **D-7950 Biberach 1 (DE)**
- **ENGELHARDT, Günther**
  **D-7950 Biberach 1 (DE)**

(56) Entgegenhaltungen:
**EP-A- 0 057 900**
**EP-A- 0 181 709**
**EP-A- 0 259 750**
**DE-A- 3 718 638**
**GB-A- 2 088 873**
**US-A- 4 119 710**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

In der EP-A-0,181,709 werden bereits 1-(4-Amino-3,5-dichlorphenyl)-2-(phenylalkoxyalkylamino)-ethanole beschrieben, welche eine stimulierende Wirkung auf die adrenergenen $\beta_2$-Rezeptoren aufweisen. Hierbei weist die Verbindung des Beispiels 4d eine geringere Wirksamkeit als die Basisverbindung 1-(4-Amino-3,5-dichlor-phenyl)-2-tert. butylamino-ethanol-hydrochlorid auf.

Außerdem werden in der US-A-4,119,710 1-(4-Amino-3-cyan-5-fluor-phenyl)-2-ethanolamine beschrieben, die am aliphatischen Stickstoffatom durch eine Cycloalkyl- oder verzweigte Alkylgruppe monosubstituiert sind. Obwohl diese Verbindungen eine große Wirkungsstärke aufweisen, sind diese auf Grund ihrer Nebenwirkungen als Therapeutica ungeeignet.

Es wurde nun gefunden, daß die Phenylethanolamine der allgemeinen Formel

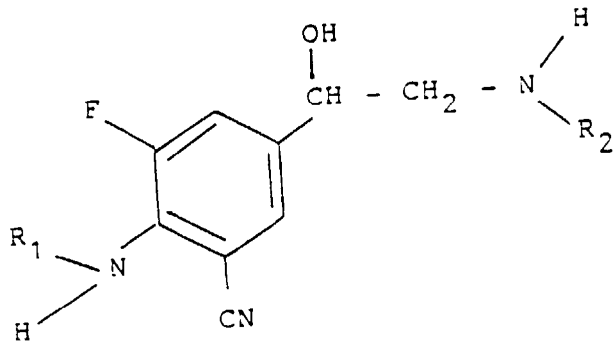

,(I)

in der

$R_1$ ein Wasserstoffatom oder eine Alkoxycarbonylgruppe mit insgesamt 2 bis 5 Kohlenstoffatomen und

$R_2$ eine Gruppe der Formel

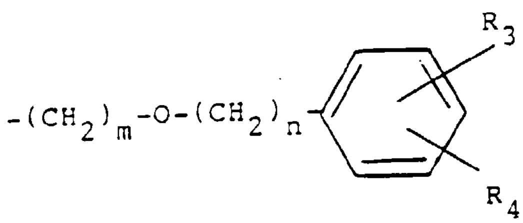

bedeuten, in der

m die Zahlen 2 bis 8,

n die Zahlen 1 bis 7,

$R_3$ und $R_4$, die gleich oder verschieden sein können, Wasserstoff-, Fluor-, Chlor- oder Bromatome, Methyl-, Ethyl-, Hydroxy-, Methoxy- oder Ethoxygruppen oder

$R_3$ und $R_4$ zusammen eine Methylendioxy- oder Ethylendioxygruppe darstellen,
deren Enantiomeren und deren Säureadditionssalze, insbesondere für die pharmazeutische Verwendung, deren physiologisch verträglichen Säureadditionssalze mit anorganischen oder organischen Säuren, wertvollerere pharmakologische Eigenschaften aufweisen, neben einer analgetischen, antiphlogistischen, broncholytischen, uterusspasmolytischen, lipolytischen und einer antispastischen Wirkung auf die quergestreifte Muskulatur weitere $\beta_2$-mimetische und/oder $\beta_1$-blockierende Wirkungen. Außerdem können diese als Leistungsförderer eingesetzt werden.

Gegenstand der vorliegenden Erfindung sind somit die Phenylethanolamine der obigen allgemeinen Formel I, deren Enantiomere, deren Säureadditionssalze, insbesondere für die pharmazeutische Verwendung deren physiologisch verträgliche Säureadditionssalze mit anorganischen oder organischen Säuren, deren Verwendung als Arzneimittel und als Leistungsförderer sowie Verfahren zu ihrer Herstellung.

Beispielsweise kommen für die bei der Definition der Reste $R_1$ und $R_2$ eingangs erwähnten Bedeutungen

für $R_1$ die des Wasserstoffatoms, der Methoxycarbonyl-, Ethoxycarbonyl-, n-Propoxycarbonyl-, Isopropoxycarbonyl-, n-Butoxycarbonyl-, 1-Methyl-n-propoxycarbonyl- oder 2-Methyl-n-propoxycarbonylgruppe und

für $R_2$ die der 2 Benzyloxy-ethyl-, 2-(2-Phenylethoxy)-ethyl-, 2-(3-Phenylpropoxy)-ethyl-, 2-(4-Phenylbutoxy)-ethyl-, 2-(5-Phenylpentoxy)-ethyl-, 2-(6-Phenylhexoxy)-ethyl-, 2-(7-Phenylheptoxy)-ethyl-, 2-(4-Methoxybenzyloxy)-ethyl-, 2-[2-(4-Methoxyphenyl)-ethoxy]-ethyl-, 2-[3-(4-Methoxyphenyl)-propoxy]-ethyl-, 2-[4-(4-Methoxyphenyl)-butoxy]-ethyl-, 2-[5-(4-Methoxyphenyl)-pentoxy]-ethyl-, 2-[6-(4-Methoxyphenyl)-hexoxy]-ethyl-, 2-[7-(4-Methoxyphenyl)-heptoxy]-ethyl-, 3-Benzyloxy-propyl-, 3-(2-Phenylethoxy)-propyl-, 3-(3-Phenylpropoxy)-propyl-, 3-(4-Phenylbutoxy)-propyl-, 3-(5-Phenylpentoxy)-propyl-, 3-(6-Phenylhexoxy)-propyl-, 3-(7-Phenylheptoxy)-propyl-, 3-(4-Methoxybenzyloxy)-propyl-, 3-[2-(4-Methoxyphenyl)-ethoxy]-propyl-, 3-[3-(4-Methoxyphenyl)-propoxy]-propyl-, 3-[4-(4-Methoxyphenyl)-butoxy]-propyl-, 3-[5-(4-Methoxyphenyl)-pentoxy]-propyl-, 3-[6-(4-Methoxyphenyl)-hexoxy]-propyl-, 3-[7-(4-Methoxyphenyl)-heptoxy]-propyl-, 4-Benzyloxy-butyl-, 4-(2-Phenylethoxy)-butyl-, 4-(3-Phenylpropoxy)-butyl-, 4-(4-Phenylbutoxy)-butyl-, 4-(5-Phenylpentoxy)-butyl-, 4-(6-Phenylhexoxy)-butyl-, 4-(7-Phenylheptoxy)-butyl-, 4-(4-Methoxybenzyloxy)-butyl-, 4-[2-(4-Methoxyphenyl)-ethoxy]-butyl-, 4-[3-(4-Methoxyphenyl)-propoxy]-butyl-, 4-[4-(4-Methoxyphenyl)-butoxy]-butyl-, 4-[5-(4-Methoxyphenyl)-pentoxy]-butyl-, 4-[6-(4-Methoxyphenyl)-hexoxy]-butyl-, 4-[7-(4-Methoxyphenyl)-heptoxy]-butyl-, 5-Benzyloxy-pentyl-, 5-(2-Phenylethoxy)-pentyl-, 5-(3-Phenylpropoxy)-pentyl-, 5-(4-Phenylbutoxy)-pentyl-, 5-(5-Phenylpentoxy)-pentyl-, 5-(6-Phenylhexoxy)-pentyl-, 5-(7-Phenylheptoxy)-pentyl-, 5-(4-Methoxybenzyloxy)-pentyl-, 5-[2-(4-Methoxyphenyl)-ethoxy]-pentyl-, 5-[3-(4-Methoxyphenyl)-propoxy]-pentyl-, 5-[4-(4-Methoxyphenyl)-butoxy]-pentyl-, 5-[5-(4-Methoxyphenyl)-pentoxy]-pentyl-, 5-[6-(4-Methoxyphenyl)-hexoxy]-pentyl-, 5-[7-(4-Methoxyphenyl)-heptoxy]-pentyl-, 6-Benzyloxy-hexyl-, 6-(2-Phenylethoxy)-hexyl-, 6-(3-Phenylpropoxy)-hexyl-, 6-(4-Phenylbutoxy)-hexyl-, 6-(5-Phenylpentoxy)-hexyl-, 6-(6-Phenylhexoxy)-hexyl-, 6-(7-Phenylheptoxy)-hexyl-, 6-(4-Methoxybenzyloxy)-hexyl-, 6-[2-(4-Methoxyphenyl)-ethoxy]-hexyl-, 6-[3-(4-Methoxyphenyl)-propoxy]-hexyl-, 6-[4-(4-Methoxyphenyl)-butoxy]-hexyl-, 6-[5-(4-Methoxyphenyl)-pentoxy]-hexyl-, 6-[6-(4-Methoxyphenyl)-hexoxy]-hexyl-, 6-[7-(4-Methoxyphenyl)-heptoxy]-hexyl-, 7-Benzyloxy-heptyl-, 7-(2-Phenylethoxy)-heptyl-, 7-(3-Phenylpropoxy)-heptyl-, 7-(4-Phenylbutoxy)-heptyl-, 7-(5-Phenylpentoxy)-heptyl-, 7-(6-Phenylhexoxy)-heptyl-, 7-(7-Phenylheptoxy)-heptyl-, 7-(4-Methoxybenzyloxy)-heptyl-, 7-[2-(4-Methoxyphenyl)-ethoxy]-heptyl-, 7-[3-(4-Methoxyphenyl)-propoxy]-heptyl-, 7-[4-(4-Methoxyphenyl)-butoxy]-heptyl-, 7-[5-(4-Methoxyphenyl)-pentoxy]-heptyl-, 7-[6-(4-Methoxyphenyl)-hexoxy]-heptyl-, 7-[7-(4-Methoxyphenyl)-heptoxy]-heptyl-, 2-(4-Chlorbenzyloxy)-ethyl-, 2-[2-(4-Chlorphenyl)-ethoxy]-ethyl-, 2-[3-(4-Chlorphenyl)-propoxy]-ethyl-, 2-[4-(4-Chlorphenyl)-butoxy]-ethyl-, 2-[5-(4-Chlorphenyl)-pentoxy]-ethyl-, 2-[6-(4-Chlorphenyl)-hexoxy]-ethyl-, 2-[7-(4-Chlorphenyl)-heptoxy]-ethyl-, 3-(4-Chlorbenzyloxy)-propyl-, 3-[2-(4-Chlorphenyl)-ethoxy]-propyl-, 3-[3-(4-Chlorphenyl)-propoxy]-propyl-, 3-[4-(4-Chlorphenyl)-butoxy]-propyl-, 3-[5-(4-Chlorphenyl)-pentoxy]-propyl-, 3-[6-(4-Chlorphenyl)-hexoxy]-propyl-, 3-[7-(4-Chlorphenyl)-heptoxy]-propyl-, 4-(4-Chlorbenzyloxy)-butyl-, 4-[2-(4-Chlorphenyl)-ethoxy]-butyl-, 4-[3-(4-Chlorphenyl)-propoxyl-butyl-, 4-[4-(4-Chlorphenyl)-butoxy]-butyl-, 4-[5-(4-Chlorphenyl)-pentoxy]-butyl-, 4-[6-(4-Chlorphenyl)-hexoxy]-butyl-, 4-[7-(4-Chlorphenyl)-heptoxy]-butyl-, 5-(4-Chlorbenzyloxy)-pentyl-, 5-[2-(4-Chlorphenyl)-ethoxy]-pentyl-, 5-[3-(4-Chlorphenyl)-propoxy]-pentyl-, 5-[4-(4-Chlorphenyl)-butoxy]-pentyl-, 5-[5-(4-Chlorphenyl)-pentoxy]-pentyl-, 5-[6-(4-Chlorphenyl)-hexoxy]-pentyl-, 5-[7-(4-Chlorphenyl)-heptoxy]-pentyl-, 6-(4-Chlorbenzyloxy)-hexyl-, 6-[2-(4-Chlorphenyl)-ethoxy]-hexyl-, 6-[3-(4-Chlorphenyl)-propoxy]-hexyl-, 6-[4-(4-Chlorphenyl)-butoxy]-hexyl-, 6-[5-(4-Chlorphenyl)-pentoxy]-hexyl-, 6-[6-(4-Chlorphenyl)-hexoxy]-hexyl-, 6-[7-(4-Chlorphenyl)-heptoxy]-hexyl-, 7-(4-Chlorbenzyloxy)-heptyl-, 7-[2-(4-Chlorphenyl)-ethoxy]-heptyl-, 7-[3-(4-Chlorphenyl)-propoxy]-heptyl-, 6- oder 7-[4-(4-Chlorphenyl)-butoxy]-heptyl-gruppe in Betracht.

Bevorzugte Verbindungen der obigen allgemeinen Formel I sind diejenigen, in denen

$R_1$ ein Wasserstoffatom, eine Methoxycarbonyl- oder Ethoxycarbonylgruppe,
m die Zahl 5, 6 oder 7,
n die Zahl 3, 4 oder 5,
$R_3$ ein Wasserstoffatom, eine Hydroxy- oder eine Methoxygruppe und
$R_4$ ein Wasserstoffatom bedeuten,

deren Enantiomere und deren Säureadditionssalze.

Die neuen Verbindungen der obigen allgemeinen Formel I lassen sich erfindungsgemäß nach folgendem Verfahren herstellen:

Reduktion eines Aldehyds der allgemeinen Formel

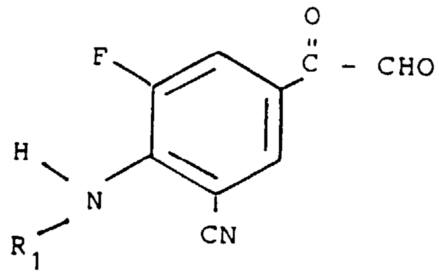

,(II)

in der
$R_1$ wie eingangs definiert ist, oder dessen Hydrats in Gegenwart eines Amins der allgemeinen Formel

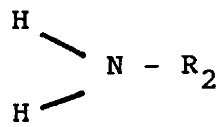

,(III)

in der
$R_2$ wie eingangs definiert ist.

Die Reduktion wird in einem Lösungsmittel wie Methanol, Äthanol, Butanol, Diäthyläther, Tetrahydrofuran oder Dioxan mit einem komplexen Metallhydrid oder mit katalytisch angeregtem Wasserstoff bei Temperaturen zwischen -20°C und der Siedetemperatur des verwendeten Lösungsmittels durchgeführt.

Zweckmäßigerweise wird beispielsweise die Reduktion mit einem komplexen Metallhydrid wie Natriumborhydrid oder Lithiumaluminiumhydrid in einem geeigneten Lösungsmittel wie Methanol, Methanol/Wasser, Diäthyläther oder Tetrahydrofuran bei Temperaturen zwischen -20°C und der Siedetemperatur des verwendeten Lösungsmittels, z.B. bei Temperaturen zwischen 0 und 50°C, und die Reduktion mit katalytisch angeregtem Wasserstoff mit Wasserstoff in Gegenwart eines Katalysators wie Platin, Palladium, Raney-Nickel oder Raney-Kobalt bei Temperaturen zwischen 0 und 100°C, vorzugsweise jedoch bei Raumtemperatur, und bei einem Wasserstoffdruck von 1-5 at durchgeführt.

Die Umsetzung wird hierbei zweckmäßigerweise in der Weise durchgeführt, daß die in situ gebildete Verbindung der allgemeinen Formel

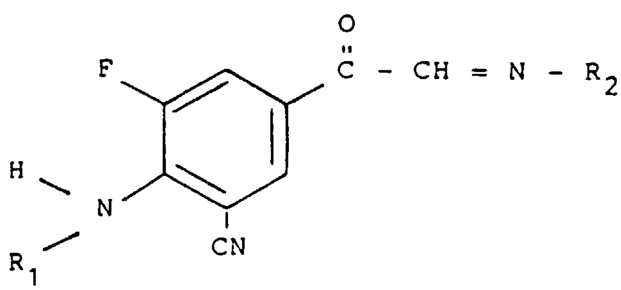

,(IV)

in der
$R_1$ und $R_2$ wie eingangs definiert sind, nicht isoliert wird, diese kann jedoch selbstverständlich isoliert und entsprechend dem oben beschriebenen Verfahren reduziert werden.

Die erhaltenen neuen Verbindungen der allgemeinen Formel I können anschließend gewünschtenfalls in ihre Enantiomere aufgetrennt werden, vorzugsweise durch fraktionierte Kristallisation eines Gemisches ihrer diastereomeren Salze mit einer optisch aktiven Säure, z.B. der D(-)-Weinsäure, L(+)-Weinsäure, Dibenzoyl-D-weinsäure, Dibenzoyl-L-weinsäure, (-)-Kampfer-10-sulfonsäure, (+)-Kampfer-10-sulfonsäure, L(-)-Äpfelsäure, D(-)-Mandelsäure, L(+)-Mandelsäure, d-α- Brom-kampfer-π-sulfonsäure oder D(-)-Chinasäure. Die Racematspaltung kann jedoch auch durch Säulenchromatographie an einem optisch aktiven Trägermaterial, z.B. an Acetylcellulose, erfolgen.

Weiterhin kann die Racematspaltung auch durch Auftrennen eines Gemisches von diastereomeren Verbindungen, die man durch Umsetzung einer Verbindung der allgemeinen Formel I mit einer chiralen Verbindung, z.B. mit einem chiralen Acylrest, wie einer N-geschützten Aminosäure, einem chiralen Kohlensäurehalbester, einer chiralen Carbonsäure oder einem chiralen Isocyanat, erhält und anschließende Abspaltung des chiralen Hilfsstoffes, durchgeführt werden. Die Auftrennung solcher diastereomerer Verbindungen erfolgt hierbei vorzugsweise durch fraktionierte Kristallisation oder durch Chromatographie an einem inerten Trägermaterial und die an-schließende Abspaltung des chiralen Hilfsstoffes zweckmäßigerweise mittels Hydrolyse oder Solvolyse.

Außerdem können die erhaltenen neuen Verbindungen der allgemeinen Formel I gewünschtenfalls mit anorganischen oder organischen Säuren in ihre physiologisch verträglichen Säureadditionssalze mit 1 Äquivalent der betreffenden Säure übergeführt werden. Als Säuren haben sich beispielsweise Salzsäure, Bromwasserstoffsäure, Schwefelsäure, Phosphorsäure, Milchsäure, Zitronensäure, Weinsäure, Maleinsäure oder Fumarsäure als geeignet erwiesen.

Die als Ausgangsstoffe verwendeten Verbindungen der allgemeinen Formeln II und III lassen sich nach an und für sich bekannten Verfahren herstellen. So erhält man beispielsweise eine Verbindung der allgemeinen Formel II durch Oxidation eines entsprechenden Acetophenons mit Selendioxid (siehe Beispiele), hierbei ist es nicht erforderlich, die benötigten Ausgangsverbindungen zu isolieren.

Wie bereits eingangs erwähnt, weisen die neuen Verbindungen der vorliegenden Anmeldung und deren physiologisch verträgliche Salze mit anorganischen und organischen Säuren bei einer guten oralen Resorption wertvolle pharmakologische Eigenschaften auf, neben einer analgetischen, antiphlogistischen, broncholytischen, uterusspasmolytischen, lipolytischen und einer antispastischen Wirkung auf die quergestreifte Muskulatur weitere $\beta_2$-mimetische und/oder $\beta_1$-blockierende Wirkungen, sie zeichnen sich insbesondere durch einen schnellen Wirkungseintritt nach oraler Gabe und einer langen Wirkungsdauer aus.

Beispielsweise wurden die Substanzen

A = 1-(4-Ethoxycarbonylamino-3-cyan-5-fluor-phenyl)-2-[6-(4-phenyl-butoxy)-hexylamino]-ethanol-hydrochlorid und

B = 1-(4-Amino-3-cyan-5-fluor-phenyl)-2-[6-(4-phenylbutoxy)-hexyl-amino]-ethanol-hydrochlorid

im Vergleich zu

V = 1-(4-Amino-3,5-dichlor-phenyl)-2-[6-(4-phenyl-butoxy)-hexylamino]-ethanol-hydrochlorid (siehe EP-A-0,181,709)

auf ihre broncholytische Wirkung wie folgt untersucht:

Die broncholytische Wirkung wurde in der Versuchsanordnung nach KONZETT und RÖSSLER (Arch. exp. Path. Pharmak. 195, 71 (1940)) an narkotisierten Meerschweinchen geprüft. Aus der mit den verschiedenen intravenösen Dosen erzielten gemittelten prozentualen Abschwächung des durch 20 µg/kg Acetylcholin i.v. ausgelösten Bronchospasmus wurde nach linearer Regressionsanalyse nach LINDER (Statistische Methoden, 4. Aufl. pp. 148-162, Birkhäuser, Basel 1964) eine $ED_{50}$ berechnet:

| Substanz | nach i.v.-Applikation |
|---|---|
| | $ED_{50}$ µg/kg |
| A | 10,20 |
| B | 2,50 |
| V | > 400 |

Die neuen Verbindungen der allgemeinen Formel sind gut verträglich, so konnte beispielsweise bei einer Applikation der Verbindungen A und B bei einer Dosis von 400 µg/kg i.v. an Meerschweinchen keine toxischen Nebenwirkungen beobachtet werden.

Die erfindungsgemäß hergestellten Verbindungen der allgemeinen Formel I und deren physiologisch verträglichen Salze mit anorganischen oder organischen Säuren eignen sich daher zur Tokolyse, zur Blutdrucksenkung durch periphere Vasodilata-tion, zur Mobilisierung von Körperfett oder zu Behandlungen von allergischen Zuständen wie allergischem Asthma oder allergischen entzündlichen Zuständen, spastischen Atemwegserkrankungen unterschiedlicher Genese oder Herzrhythmusstörungen, und lassen sich hierzu, gegebenenfalls in Kombination mit anderen Wirksubstanzen, in die übliche pharmazeutischen Zubereitungsformen wie Tabletten, Dragees, Lösungen, Sprays, Ampullen oder Suppositorien einarbeiten. Die Einzeldosis beträgt hierbei am Menschen 1 bis 50 µg, vorzugsweise 2,5 bis 25 µg, ein- bis zweimal täglich.

Desweiteren können die neuen Verbindungen der allgemeinen Formel I und deren Säureadditionssalze zur Behandlung fettsüchtiger Tiere wie Hunde und als Folge ihrer körperfettreduzierenden (lipolytischen) Wirkung zur Reduktion unerwünschter Fetteinlagerungen bei der Mastzucht, also zur Verbesserung der Fleischqualität von Masttieren wie Schweinen, Rindern, Schafen und Geflügel eingesetzt werden. Bei den Tieren kann die Applikation der oben genannten Verbindungen oral oder auch nicht-oral, z.B. als Futterzusatz oder durch Injektion oder auch über implantierte Minipumpen erfolgen. Die Tagesdosis liegt hierbei zwischen 0,01 und 100 µg/kg, vorzugsweise jedoch zwischen 0,01 bis 10 µg/kg Körpergewicht.

Desweiteren können die neuen Verbindungen der allgemeinen Formel I und deren Säureadditionssalze als Leistungsförderer bei Tieren zur Förderung und Beschleunigung des Wachstums, der Milch- und Wollproduktion, sowie zur Verbesserung der Futterverwertung, der Schlachtkörperqualität und zur Verschiebung des Fleisch-Fett-Verhältnisses zugunsten von Fleisch eingesetzt werden. Die Wirkstoffe werden bei Nutz-, Zucht-, Zier- und Hobbytieren verwendet.

Zu den Nutz- und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänse, Enten, Truthühner, Fische, wie z. B. Karpfen, Forellen, Lachse, Aale, Schleien, Hechte, Reptilien wie z.B. Schlangen und Krokodile.

Zu den Zier- und Hobbytieren zählen Säugetiere wie Hunde und Katzen, Vögel wie Papageien, Kanarienvögel, Fische wie Zier- und Aquarienfische, z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichtung des gewünschten Effektes verabreicht wird, kann wegen der günstigen Eigenschaften der Wirkstoffe weitgehend variiert werden. Sie liegt vorzugsweise bei etwa 0,01 bis 50 µg/kg, insbesondere bei 0,01 bis 25 µg/kg Körpergewicht pro Tag.

Die passende Menge des Wirkstoffs sowie die passende Dauer der Verabreichung hängen insbesondere von der Tierart, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Tierart, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase temporär oder kontinuierlich verabreicht werden. Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form. Orale Formulierungen sind Pulver, Tabletten, Granulate, Drenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppb - 100 %, bevorzugt von 0,01 ppb - 10 %. Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Konzentration der Wirkstoffe im Fertigfutter beträgt normalerweise etwa 0,01 ppb - 50 ppm, bevorzugt 0,1 ppb - 10 ppm.

Die Wirkstoffe können als solche oder in Form von Prämixen oder Futterkonzentraten dem Futter zugesetzt werden.

So enthalten die erfindungsgemäßen Futtermittel neben dem Wirkstoff und gegebenenfalls neben einer üblichen Vitamin-Mineral-Mischung beispielsweise für Mastschweine Gerste, Weizennachmehl, Ackerbohnen, Raps-Extraktionsschrot und Futterfett, für Broiler Mais, Sojabohnenmehl, Fleischmehl, Futterfett und Sojaöl, für Rinder Zuckerrübenschnitzel Maiskleber, Malzkeime, Sojabohnenmehl, Weizen und Molasse sowie für Lämmer Gerste, Sojabohnenmehl, Mais und Melasse. Zu diesem Futter wird eine der eingangs erwähnten Verbindungen der Formel I als Wirkstoff in einer Konzentration von 0,01 ppb bis 0,50 %, vorzugsweise jedoch von 0,1 ppb bis 0,05 %, zugemischt, wobei die Zumischung vorzugsweise in Form einer Wirkstoffvormischung erfolgt. Diese Vormischung enthält beispielsweise 5 bis 10 000 mg, vorzugsweise jedoch 50 bis 1 000 mg, zweckmäßigerweise in 1 000 g Maisstärke.

Die nachfolgenden Beispiele sollen die Erfindung näher erläutern:

Beispiel A

6-(4-Phenylbutoxy)-hexylamin

Eine Lösung von 46,6 g (0,15 Mol) 6-(4-Phenylbutoxy)-hexylbromid und 27,6 g (0,15 Mol) Phthalimidkalium in 400 ml Aceton wird während 70 Stunden auf Rückflußtemperatur erhitzt. Nach dem Abkühlen saugt man vom ausgeschiedenen Kaliumbromid ab und destilliert das Lösungsmittel unter Vakuum ab. Der so erhaltene ölige Rückstand, bestehend aus rohem 6-(4-Phenylbutoxy)-N-hexyl-phthalimid, wird in 300 ml Dichlormethan und 300 ml 40%iger Methylaminlösung über Nacht gerührt. Die organische Phase wird abgetrennt und die wässrige Phase noch zweimal mit Dichlormethan extrahiert. Die vereinigten organischen Phasen werden über Natriumsulfat getrocknet und im Vakuum eingeengt. Der ölige Rückstand wird in Äther gelöst, die sich ausscheidenden Festkörper werden abfiltriert und das Filtrat wird zur Trockne eingedampft. Das verbleibende farblose Öl wird destilliert.
$K_{p\ 0,2}$ - 143-145°C

Beispiel 1

1-(4-Ethoxycarbonylamino-3-cyan-5-fluor-phenyl)-2-[6-(4-phenyl-butoxy)-hexylamino]-ethanol-hydrochlorid

In eine Lösung von 2,4 g Selendioxid in 50 ml Dioxan und 3 ml Wasser werden bei 60°C unter Rühren 5 g (0,02 Mol) 4'-Ethoxycarbonylamino-3'-cyan-5'-fluor-acetophenon und 1,5 g Kieselgur eingetragen. Anschließend wird 4 Stunden lang auf Rückflußtemperatur erhitzt und anschließend vom Festkörper abfiltriert. Zu der so erhaltenen Lösung von 4'-Ethoxycarbonylamino-3'-cyan-5'-fluor-phenylglyoxal werden nach Abkühlen 7 g (0,025 Mol) 6-(4-Phenylbutoxy)-hexylamin, gelöst in wenig Dioxan, gegeben. Nach einer Stunde Stehen bei Raumtemperatur verdünnt man mit 100 ml Äthanol und trägt unter Rühren und Kühlen 3 g Natriumborhydrid ein. Man läßt über Nacht bei Raumtemperatur stehen und zerstört überschüssiges Natriumborhydrid mit Aceton und engt anschließend im Vakuum zur Trockne ein. Der feste Rückstand wird zwischen Wasser und Dichlormethan verteilt und das Ungelöste über Kieselgur abgesaugt. Der Filterkuchen wird noch dreimal mit Dichlormethan gewaschen. Die vereinigten Dichlormethan-Lösungen werden getrocknet und im Vakuum zur Trockne eingeengt. Der feste Rückstand wird mittels Chromatographie über Kieselgel gereinigt, wobei Dichlormethan/Methanol = 20/1 und 8/1 als Elutionsmittel dient. Der nach dem Einengen erhaltene Rückstand wird in Äthanol gelöst. Diese Lösung säuert man mittels ätherischer Salzsäure bis pH 3 an. Nach Zugabe von Äther tritt Kristallisation ein.
Schmelzpunkt: 119-123°C

Beispiel 2

1-(4-Amino-3-cyan-5-fluor-phenyl)-2-[6-(4-phenylbutoxy)-hexylamino]-ethanol-hydrochlorid

Hergestellt analog Beispiel 1, ausgehend von 4'-Amino-3'cyan-5'-fluor-acetophenon und 6-(4-Phenylbutoxy)-hexylamin. Schmelzpunkt: 173-176°C

Beispiel 3

Tabletten mit 5 µg 1-(4-Ethoxycarbonylamino-3-cyan-5-fluorphenyl)-2-[6-(4-phenyl-butoxy)-hexylamino]-ethanol-hydrochlorid

Zusammensetzung:

1 Tablette enthält:

| | |
|---|---|
| Wirksubstanz | 0,005 mg |
| Milchzucker | 82,495 mg |
| Kartoffelstärke | 33,000 mg |
| Polyvinylpyrrolidon | 4,000 mg |
| Magnesiumstearat | 0,500 mg |
| | 120,000 mg |

Herstellungsverfahren:

Die Wirksubstanz und Polyvinylpyrrolidon werden in Äthanol gelöst. Die Mischung von Milchzucker und Kartoffelstärke wird mit der Wirkstoff-/Granulierlösung gleichmäßig befeuchtet. Die Feuchtsiebung erfolgt mit 1,5 mm-Maschenweite. Anschließend wird bei 50°C getrocknet und die Trockensiebung mit 1,0 mm-Maschenweite vorgenommen. Das so erhaltene Granulat wird mit Magnesiumstearat gemischt und zu Tabletten verpreßt.

| | |
|---|---|
| Tablettengewicht: | 120 mg |
| Stempel: | 7 mm, flach |

Beispiel 4

Gelatine-Steckkapseln mit 5 µg 1-(4-Ethoxycarbonylamino-3-cyan-5-fluor-phenyl)-2-[6-(4-phenyl-butoxy)-hexylamino]-ethanol-hydrochlorid

Zusammensetzung:

1 Tablette enthält:

| | |
|---|---|
| Wirksubstanz | 0,005 mg |
| Milchzucker | 59,995 mg |
| Maisstärke | 60,000 mg |
| | 120,000 mg |

Herstellungsverfahren:

Die Wirksubstanz wird mit Milchzucker und Maisstärke intensiv gemischt und in Gelatine-Steckkapseln geeigneter Größe abgefüllt.

Kapselfüllung: 120,0 mg

Beispiel 5

Ampullen mit 2 µg 1-(4-Amino-3-cyan-5-fluor-phenyl)-2-[6-(4-phenylbutoxy)-hexyl-amino]-ethanol-hydrochlorid pro 2 ml

Zusammensetzung:

1 Ampulle enthält:

| | | |
|---|---|---|
| Wirksubstanz | | 0,002 mg |
| Zitronensäure | | 2,500 mg |
| Natriumhydrogenphosphat | | 7,500 mg |
| Kochsalz | | 4,600 mg |
| Ampullenwasser | ad | 2,000 ml |

Herstellungsverfahren:

Die Wirksubstanz, Puffersubstanzen und Kochsalz werden in Ampullenwasser gelöst und anschließend keimfrei filtriert.

| | |
|---|---|
| Abfüllung: | in braune Ampullen zu 2 ml unter Schutzbegasung ($N_2$) |
| Sterilisation: | 20 Minuten bei 120°C |

Beispiel 6

Sirup mit 2 µg 1-(4-Ethoxycarbonylamino-3-cyan-5-fluor-phenyl)-2-[6-(4-phenyl-butoxy)-hexylamino]-ethanol-hydrochlorid pro 5 ml

Zusammensetzung:

100 ml Sirup enthält:

| | |
|---|---|
| Wirksubstanz | 0,04 mg |
| Benzoesäure | 0,10 g |
| Weinsäure | 1,00 g |
| Zucker | 50,00 g |
| Apfelsinen-Aroma | 1,00 g |
| Lebensmittelrot | 0,05 g |
| Dest. Wasser ad | 100,00 ml |

Herstellungsverfahren:

Ca. 60 g dest. Wasser werden auf 80°C erwärmt und darin nacheinander Benzoesäure, Weinsäure, die Wirksubstanz, der Farbstoff und Zucker gelöst. Nach Abkühlung auf Raumtemperatur wird das Aroma zugegeben und auf das gegebene Volumen aufgefüllt. Der Sirup wird filtriert.

Beispiel 7

Aerosol-Spray mit 1 µg 1-(4-Amino-3-cyan-5-fluor-phenyl)-2-[6-(4-phenylbutoxy)-hexyl-amino]-ethanol-hydrochlorid pro Hub

Zusammensetzung:

| | |
|---|---|
| Wirksubstanz | 0,00025 mg |
| Ethanol rein 99,9%ig | 0,87500 mg |
| Treibgasgemisch 11/12/114 (23:54:23) | 69,12475 mg |
| | 70,00000 mg |

Beispiel 8

Inhalationslösung mit 59 mg 1-(4-Amino-3-cyan-5-fluor-phenyl)-2-[6-(4-phenylbutoxy)-hexyl-amino]-ethanol-hydrochlorid pro 100 ml

Zusammensetzung:

| | |
|---|---|
| Wirksubstanz | 0,59 mg |
| Natriumchlorid | 900,00 mg |
| Benzalkoniumchlorid | 25,00 mg |
| Dest. Wasser ad | 100,00 ml |

Herstellungsverfahren:

Die Wirksubstanz, Kochsalz und Benzalkoniumchlorid werden in dest. Wasser gelöst und anschließend keimfrei filtriert.

Beispiel 9

Alleinfutter II für Mastschweine

| | |
|---|---|
| Gerste | 379 g/kg |
| Weizennachmehl | 200 g/kg |
| Maniokmehl | 135 g/kg |
| Ackerbohnen | 100 g/kg |
| Raps-Extraktionsschrot | 100 g/kg |
| Futterfett | 65 g/kg |
| lysinreiches Mineralfutter für Schweine | 20 g/kg |
| Wirkstoff-Vormischung | 1 g/kg |

Diese Komponenten in den angegebenen Mengen ergeben nach sorgfältigem Mischen 1 kg Futter.
Die 1 g Wirkstoffvormischung enthalten z.B. 2 mg einer erfindungsgemäßen Verbindung und 0,998 g Maisstärke.

Beispiel 10

Mastfutter II für Broiler

| | |
|---|---|
| Mais | 634 g/kg |
| Sojabohnenmehl | 260 g/kg |
| Fleischmehl | 40 g/kg |
| Futterfett | 25 g/kg |
| Sojaöl | 17 g/kg |
| Bicalciumphosphat | 12 g/kg |
| Calciumcarbonat | 6 g/kg |
| Vitamin-/Mineralstoffmischung | 5 g/kg |
| Wirkstoff-Vormischung | 1 g/kg |

Diese Komponenten in den angegebenen Mengen ergeben nach sorgfältigem Mischen 1 kg Futter.
Die 1 g Wirkstoffvormischung enthalten z.B. 1 mg einer erfindungsgemäßen Verbindung und 0,999 g Maisstärke.

Beispiel 11

Kraftfutter für Rinder

| | |
|---|---|
| Zuckerrübenschnitzel | 600,0 g/kg |
| Maiskleber | 100,0 g/kg |
| Malzkeime | 50,0 g/kg |
| Sojabohnenmehl | 35,0 g/kg |
| Weizen | 119,0 g/kg |
| Melasse | 60,0 g/kg |
| Futterphosphate | 12,0 g/kg |
| Calciumcarbonat | 2,5 g/kg |
| Salz | 5,0 g/kg |
| Mineralstoffe | 10,0 g/kg |
| Vitamin-Vormischung | 5,5 g/kg |
| Wirkstoff-Vormischung | 1,0 g/kg |

Diese Komponenten in den angegebenen Mengen ergeben nach sorgfältigem Mischen 1 kg Futter.

Die 1 g Wirkstoffvormischung enthalten z.B. 2 mg einer erfindungsgemäßen Verbindung und 0,998 g Maisstärke.

Beispiel 12

Mastfutter für Lämmer

| | |
|---|---|
| Gerste | 690 g/kg |
| Sojabohnenmehl | 100 g/kg |
| Mais | 159 g/kg |
| Melasse | 30 g/kg |
| Vitamin-/Mineralstoffmischung | 20 g/kg |
| Wirkstoffvormischung | 1 g/kg |

Diese Komponenten in den angegebenen Mengen ergeben nach sorgfältigem Mischen 1 kg Futter.
Die 1 g Wirkstoffvormischung enthalten z.B. 2 mg Wirkstoff und 0,998 g Maisstärke.

**Patentansprüche**

**1.** Phenylethanolamine der allgemeinen Formel

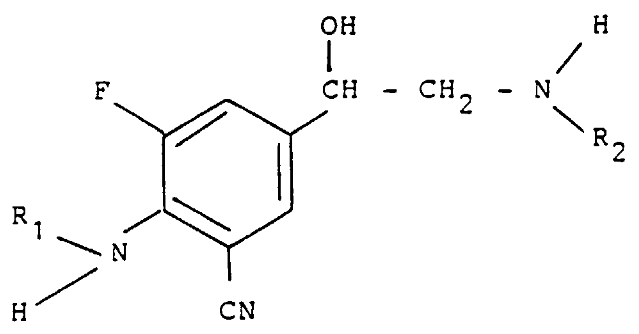

,(I)

in der

$R_1$ ein Wasserstoffatom oder eine Alkoxycarbonylgruppe mit insgesamt 2 bis 5 Kohlenstoffatomen und

$R_2$ eine Gruppe der Formel

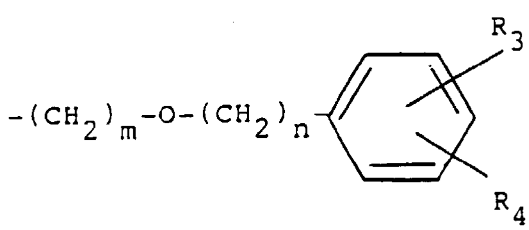

bedeuten, in der

m die Zahlen 2 bis 8,

n die Zahlen 1 bis 7,

$R_3$ und $R_4$, die gleich oder verschieden sein können, Was-serstoff-, Fluor-, Chlor- oder Bromatome, Methyl-, Ethyl-, Hydroxy-, Methoxy- oder Ethoxygruppen oder

$R_3$ und $R_4$ zusammen eine Methylendioxy- oder Ethylendioxygruppe darstellen, deren Enantiomere und deren Säureadditionssalze.

**2.** Phenylethanolamine der allgemeinen Formel I gemäß Anspruch 1, in der

$R_1$ ein Wasserstoffatom, eine Methoxycarbonyl- oder Ethoxycarbonylgruppe,
m die Zahl 5, 6 oder 7,
n die Zahl 3, 4 oder 5,
$R_3$ ein Wasserstoffatom, eine Hydroxy- oder eine Methoxygruppe und
$R_4$ ein Wasserstoffatom bedeuten, deren Enantiomere und deren Säureadditionssalze.

**3.** 1-(4-Ethoxycarbonylamino-3-cyan-5-fluor-phenyl)-2-[6-(4-phenyl-butoxy)-hexylamino]-ethanol, dessen Enantiomere und dessen Säureadditionssalze.

**4.** 1-(4-Amino-3-cyan-5-fluor-phenyl)-2-[6-(4-phenylbutoxy)-hexyl-amino]-ethanol, dessen Enantiomere und dessen Säureadditionssalze.

**5.** Physiologisch verträgliche Säureadditionssalze der Verbindung gemäß den Ansprüchen 1 bis 4.

**6.** Arzneimittel, enthaltend eine Verbindung gemäß den Ansprüchen 1 bis 4 oder ein physiologisch verträgliches Säureadditionssalz gemäß Anspruch 5 neben gegebenenfalls einem oder mehreren inerten Trägerstoffen und/oder Verdünnungsmitteln.

**7.** Verwendung einer Verbindung gemäß den Ansprüchen 1 bis 5 zur Herstellung eines Arzneimittels, das für die Behandlung der Fettsucht, von obstruktiven Lungenveränderungen, allergischem Asthma-Bronchiale, spastischen Bronchitiden, Entzündungen oder vorzeitiger Wehentätigkeit geeignet ist.

**8.** Verfahren zur Herstellung eines Arzneimittels gemäß Anspruch 7, dadurch gekennzeichnet, daß auf nichtchemischem Wege eine Verbindung gemäß den Ansprüchen 1 bis 5 in einen oder mehrere inerte Trägerstoffe und/oder Verdünnungsmittel eingearbeitet wird.

**9.** Verwendung einer Verbindung gemäß den Ansprüchen 1 bis 5 als leistungsförderndes Mittel bei Tieren.

**10.** Tiernahrung und Prämixe zur Herstellung von Tiernahrung, enthaltend eine Verbindung gemäß den Ansprüchen 1 bis 5.

**11.** Leistungsförderndes Mittel für Tiere, enthaltend eine Verbindung gemäß den Ansprüchen 1 bis 5.

**12.** Verfahren zur Herstellung von leistungsfördernden Mitteln für Tiere, dadurch gekennzeichnet, daß eine Verbindung gemäß den Ansprüchen 1 bis 5 mit Streck-, Verdünnungs- und Nahrungsmitteln sowie gegebenenfalls weiteren Hilfsstoffen vermischt wird.

**13.** Verfahren zur Herstellung der Verbindungen gemäß den Ansprüchen 1 bis 5, dadurch gekennzeichnet, daß ein Aldehyd der allgemeinen Formel

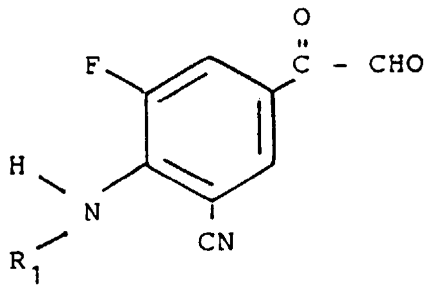

,(II)

in der
$R_1$ wie in den Ansprüchen 1 bis 3 definiert ist, oder dessen Hydrat in Gegenwart eines Amins der allgemeinen Formel

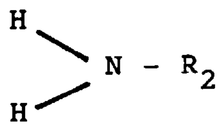

,(III)

in der

$R_2$ wie in den Ansprüchen 1 bis 3 definiert ist, reduziert wird und

gewünschtenfalls anschließend ein so erhaltenes Racemat in ihre Enantiomeren aufgetrennt wird oder

eine so erhaltene Verbindung der allgemeinen Formel I in ihre Säureadditionssalze, insbesondere für die pharmazeutische Anwendung in ihre physiologisch verträglichen Säureadditions-salze mit anorganischen oder organischen Säuren, überführt wird.

**14.** Verfahren gemäß Anspruch 13, dadurch gekennzeichnet, daß eine in situ hergestellte Verbindung der allgemeinen Formel

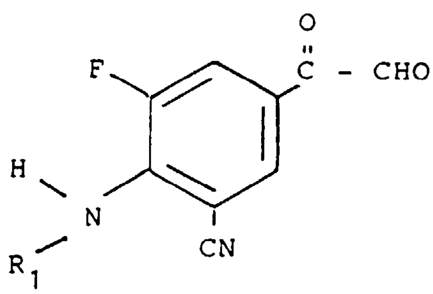

,(II)

in der
$R_1$ und $R_2$ wie in den Ansprüchen 1 bis 3 definiert sind, reduziert wird.

**15.** Verfahren gemäß den Ansprüchen 13 und 14, dadurch gekennzeichnet, daß die Racematspaltung durch fraktionierte Kristallisation oder durch Chromatographie eines diastereomeren Salzes einer Verbindung der allgemeinen Formel I durchgeführt wird.

**16.** Verfahren gemäß den Ansprüchen 13 und 14, dadurch gekennzeichnet, daß die Racematspaltung durch fraktionierte Kristallisation oder durch Chromatographie diastereomerer Verbindungen, welche durch Umsetzung einer Verbindung der Formel I mit einem chiralen Hilfsstoff erhalten werden, durchgeführt und anschließend der chirale

Hilfsstoff wieder abgespalten wird.

## Claims

1. Phenylethanolamines of general formula

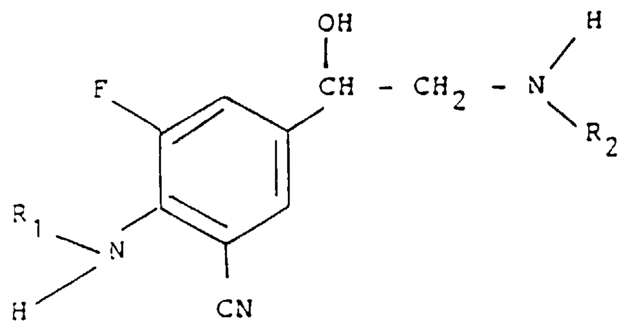

, (I)

wherein

$R_1$ represents a hydrogen atom or an alkoxycarbonyl group having a total of 2 to 5 carbon atoms and

$R_2$ represents a group of the formula

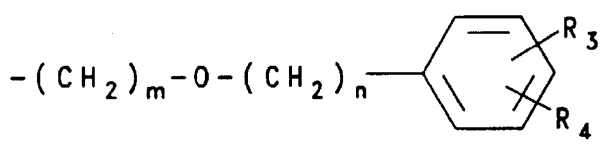

wherein

m represents the numbers 2 to 8,
n represents the numbers 1 to 7,
$R_3$ and $R_4$, which may be identical or different, represent hydrogen, fluorine, chlorine or bromine atoms, methyl, ethyl, hydroxy, methoxy or ethoxy groups or

$R_3$ and $R_4$ together represent a methylenedioxy or ethylenedioxy group,

the enantiomers and the acid addition salts thereof.

2. Phenylethanolamines of general formula I according to claim 1, wherein

$R_1$ represents a hydrogen atom, a methoxycarbonyl or ethoxycarbonyl group,
m represents the number 5, 6 or 7,
n represents the number 3, 4 or 5,
$R_3$ represents a hydrogen atom, a hydroxy or methoxy group, and
$R_4$ represents a hydrogen atom,

the enantiomers and the acid addition salts thereof.

3. 1-(4-ethoxycarbonylamino-3-cyano-5-fluoro-phenyl)-2-[6-(4-phenylbutoxy)-hexylamino]-ethanol, the enantiomers and the acid addition salts thereof.

4. 1-(4-amino-3-cyano-5-fluoro-phenyl)-2-[6-(4-phenylbutoxy)-hexylamino]-ethanol, the enantiomers and the acid

addition salts thereof.

**5.** Physiologically acceptable acid addition salts of the compounds according to claims 1 to 4.

**6.** Pharmaceutical compositions containing a compound according to claims 1 to 4 or a physiologically acceptable acid addition salt according to claim 5, optionally together with one or more inert carriers and/or diluents.

**7.** Use of a compound according to claims 1 to 5 for preparing a pharmaceutical composition suitable for treating obesity, obstructive changes in the lungs, allergic bronchial asthma, spastic bronchitis, inflammation or premature labour.

**8.** Process for preparing a pharmaceutical composition according to claim 7, characterised in that a compound according to claims 1 to 5 is incorporated in one or more inert carriers and/or diluents by a non-chemical method.

**9.** Use of a compound according to claims 1 to 5 as a performance enhancer in animals.

**10.** Animal feed and premixes for preparing animal feed, containing a compound according to claims 1 to 5.

**11.** Performance-enhancing agent for animals, containing a compound according to claims 1 to 5.

**12.** Process for preparing performance-enhancing agents for animals, characterised in that a compound according to claims 1 to 5 is mixed with extenders, diluents and feedstuffs and optionally other adjuvants.

**13.** Process for preparing the compounds according to claims 1 to 5, characterised in that an aldehyde of general formula

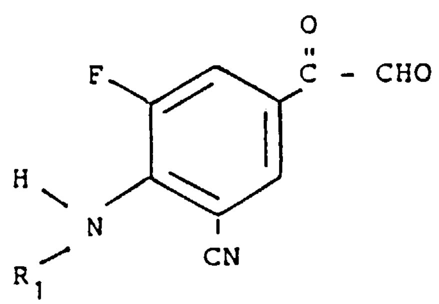

,(II)

(wherein

$R_1$ is defined as in claims 1 to 3) or a hydrate thereof, is reduced in the presence of an amine of general formula

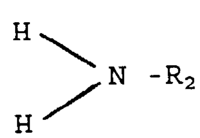

(III)

wherein

$R_2$ is defined as in claims 1 to 3, and

subsequently, if desired, a racemate thus obtained is resolved into the enantiomers thereof, or

a compound of general formula I thus obtained is converted into the acid addition salts thereof, more particularly, for pharmaceutical use, into the physiologically acceptable acid addition salts thereof with inorganic or organic acids.

**14.** Process according to claim 13, characterised in that a compound of general formula

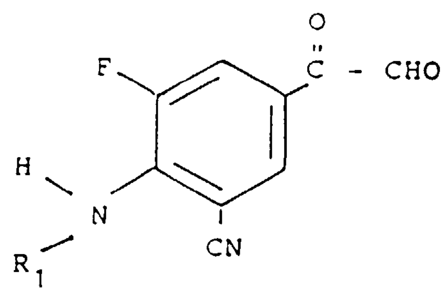

, (II)

produced iu situ, wherein
$R_1$ and $R_2$ are defined as in claims 1 to 3, is reduced.

**15.** Process according to claims 13 and 14,
characterised in that the racemate separation is carried out by fractional crystallisation or by chromatography of a diastereomeric salt of a compound of general formula I.

**16.** Process according to claims 13 and 14,
characterised in that the racemate separation is carried out by fractional crystallisation or by chromatography of diastereomeric compounds obtained by reacting a compound of formula I with a chiral adjuvant, and then the chiral adjuvant is split off again.

**Revendications**

**1.** Phényléthanolamines de formule générale

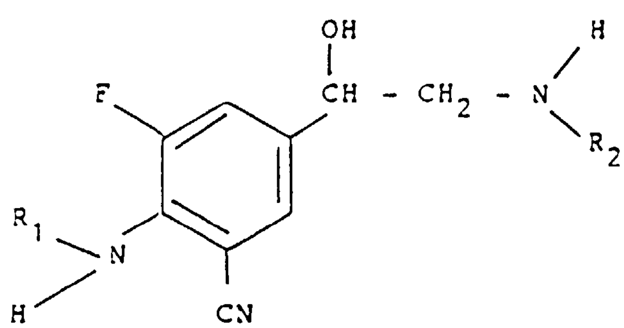

(I)

dans laquelle

$R_1$ représente un atome d'hydrogène ou un groupe alcoxycarbonyle ayant au total 2 à 5 atomes de carbone et
$R_2$ représente un groupe de formule

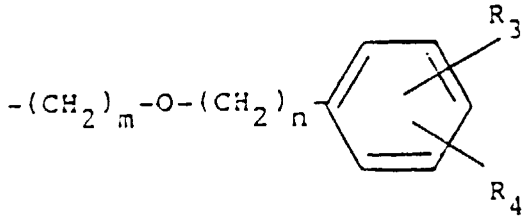

dans laquelle

m représente les nombres 2 à 8,
n représente les nombres 1 à 7,
$R_3$ et $R_4$, qui peuvent être identiques ou différents, représentent des atomes d'hydrogène, de fluor, de chlore ou de brome, des groupes méthyle, éthyle, hydroxyle, méthoxy ou éthoxy ou
$R_3$ et $R_4$ représentent ensemble un groupe méthylènedioxy ou éthylènedioxy, leurs énantiomères et leurs sels d'addition d'acide.

**2.** Phényléthanolamines de formule générale I selon la revendication 1, dans laquelle

$R_1$ représente un atome d'hydrogène, un groupe méthoxycarbonyle ou éthoxycarbonyle,
m représente le nombre 5, 6 ou 7,
n représente le nombre 3, 4 ou 5,
$R_3$ représente un atome d'hydrogène, un groupe hydroxyle ou un groupe méthoxy et
$R_4$ représente un atome d'hydrogène, leurs énantiomères et leurs sels d'addition d'acide.

**3.** 1-(4-éthoxycarbonylamino-3-cyano-5-fluorophényl)-2-[6-(4-phénylbutoxy)hexylamino]éthanol, ses énantiomères et ses sels d'addition d'acide.

**4.** 1-(4-amino-3-cyano-5-fluorophényl)-2-[6-(4-phényl-butoxy)hexylamino]éthanol, ses énantiomères et ses sels d'addition d'acide.

**5.** Sels d'addition d'acide physiologiquement acceptables du composé selon les revendications 1 à 4.

**6.** Médicament contenant un composé selon les revendications 1 à 4 ou un sel d'addition d'acide physiologiquement acceptable selon la revendication 5 et éventuellement un ou plusieurs supports et/ou diluants inertes.

**7.** Utilisation d'un composé selon les revendications 1 à 5 pour la préparation d'un médicament qui convient pour le traitement de l'adipose, des modifications obstructives des poumons, de l'asthme bronchique allergique, des bronchites spasmodiques, des inflammations ou des douleurs prématurées.

**8.** Procédé de préparation d'un médicament selon la revendication 7 caractérisé en ce que, par voie non chimique, un composé selon les revendications 1 à 5 est incorporé dans un ou plusieurs supports et/ou diluants inertes.

**9.** Utilisation d'un composé selon les revendications 1 à 5 comme agent améliorant les performances chez les animaux.

**10.** Aliment pour animaux et prémélanges pour la production d'un aliment pour animaux contenant un composé selon les revendications 1 à 5.

**11.** Agent améliorant les performances pour animaux contenant un composé selon les revendications 1 à 5.

**12.** Procédé de préparation d'agents améliorant les performances pour animaux caractérisé en ce qu'un composé selon les revendications 1 à 5 est mélangé avec des agents de délayage, des diluants et des produits alimentaires et éventuellement avec d'autres substances auxiliaires.

**13.** Procédé de préparation des composés selon les revendications 1 à 5 caractérisé en ce qu'un aldéhyde de formule générale

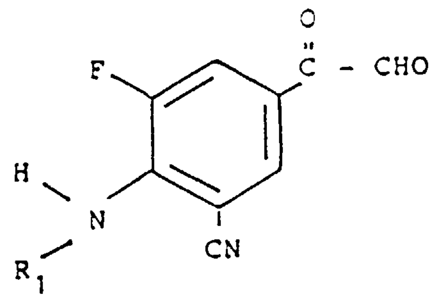

(II)

dans laquelle

$R_1$ est défini comme dans les revendications 1 à 3, ou son hydrate, est réduit en présence d'une amine de formule générale

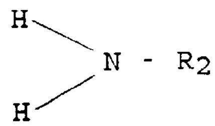

(III)

dans laquelle

$R_2$ est défini comme dans les revendications 1 à 3, puis, si on le souhaite, un racémique ainsi obtenu est dédoublé en ses énantiomères ou

un composé de formule générale I ainsi obtenu est converti en ses sels d'addition d'acide, en particulier pour l'utilisation pharmaceutique en ses sels d'addition d'acide physiologiquement acceptables avec des acides inorganiques ou organiques.

**14.** Procédé selon la revendication 13 caractérisé en ce qu'un composé préparé in situ de formule générale

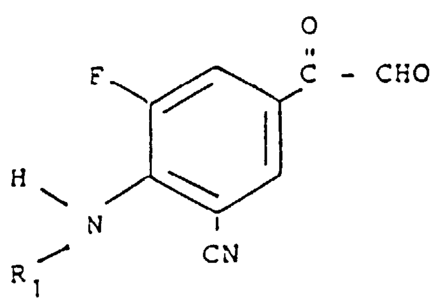

(II)

dans laquelle
$R_1$ et $R_2$ sont définis comme dans les revendications 1 à 3 est réduit.

**15.** Procédé selon les revendications 13 et 14 caractérisé en ce que le dédoublement de racémique est réalisé par cristallisation fractionnée ou par chromatographie d'un sel diastéréoisomère d'un composé de formule générale I.

**16.** Procédé selon les revendications 13 et 14 caractérisé en ce que le dédoublement de racémique est réalisé par cristallisation fractionnée ou par chromatographie de composés diastéréoisomères qui sont obtenus par réaction d'un composé de formule I avec une substance auxiliaire chirale après quoi la substance auxiliaire chirale est clivée de nouveau.